Europäisches Patentamt

European Patent Office

Office européen des brevets

(11) Publication number: **0 239 405**
**A2**

(12) **EUROPEAN PATENT APPLICATION**

(21) Application number: **87302640.5**

(22) Date of filing: **26.03.87**

(51) Int. Cl.⁴: **C 12 P 21/00**

(30) Priority: **28.03.86 US 845899**

(43) Date of publication of application:
**30.09.87 Bulletin 87/40**

(84) Designated Contracting States:
**AT BE CH DE ES FR GB GR IT LI LU NL SE**

(71) Applicant: **THE REGENTS OF THE UNIVERSITY OF CALIFORNIA**
**2199 Addison Street**
**Berkeley California 94720 (US)**

(72) Inventor: **Vanderlaan, Martin**
**2764 Bolinger Canyon Road**
**San Ramon California 94583 (US)**

**Thomas, Cynthia B.**
**2390 Pasateimpo Street**
**Livermore California 94550 (US)**

**Watkins, Bruce E.**
**3998 East Avenue Apartment K.**
**Livermore California 94550 (US)**

**Stanker, Larry H.**
**653 Anna Maria Street**
**Livermore California 94550 (US)**

(74) Representative: **Goldin, Douglas Michael et al**
**J.A. KEMP & CO. 14, South Square Gray's Inn**
**London WC1R 5EU (GB)**

The microorganism(s) has (have) been deposited with ATCC under number(s) HB 8916.

(54) **Monoclonal antibodies against halodeoxyuridines and hybridoma producing the same.**

(57) Monoclonal antibodies specific for halodeoxyuridines incorporated into DNA, and a hybridoma producing these antibodies are described. The affinity of the monoclonal antibodies does not depend on the density of incorporated halodeoxyuridine, and it is at least an order of magnitude better than currently available antibodies. The monoclonal antibodies of the invention have direct applications in assays for DNA synthesis, and for monitoring the cytotoxicity of S phase specific anti-cancer agents.

0 239 405

## Description

MONOCLONAL ANTIBODIES AGAINST HALODEOXYURIDINES AND HYBRIDOMA PRODUCING THE SAME

The United States Government has rights in this invention pursuant to Contract No. w-7405-ENG-48 between the U.S. Department of Energy and the University of California, for the operation of Lawrence Livermore National Laboratory.

BACKGROUND OF THE INVENTION

The invention relates generally to monoclonal antibodies and associated hybridomas, and more particularly to monoclonal antibodies against halodeoxyuridines incorporated in DNA.

A range of biomedical investigations depends on the ability to identify and quantify DNA-synthesizing, or S-phase, cells. Oncologists, for example, have devoted substantial effort to establishing correlations between the fraction of tumor cells synthesizing DNA and treatment prognosis, e.g. Hart et al., Cancer, Vol. 39, pgs. I603-I607 (I977). Effort has also been devoted to improvement of cancer therapy with S-phase specific anti-cancer agents by treating when the experimentally determined frequency of tumor cells in S phase is maximal, Barranco, et al., Cancer Research, Vol. 42, pgs. 2894-2898 (I982). And finally, many assays for monitoring the effectiveness of chemotherapeutic drugs are based on in vitro detection of proliferating tumor cells, e.g. Roobol et al., J. Nat. Cancer Inst. Vol. 72, pgs. 66I-668 (I984); Solomon et al., U. S. Patent 4,4II,990; and waldman et al; Cytometry, Vol. 6, pgs. 657-662 (I985).

A standard procedure for detecting replicating cell populations and assaying for DNA synthesis includes growing cells in a culture media in which one or more of the nucleoside precursors of DNA (thymidine, deoxycytidine, deoxyadenosine, or deoxyguanosine) are replaced by radioactively labeled forms, or otherwise detectable analogs, e.g. Gray et al., Cell and Tissue Kinetics, Vol. IO, pgs. 97-IO9 (I977); Steel, Cell and Tissue Kinetics, Vol. 5, pg. 87 (I972); Gratzner, Science, Vol. 2I8, pgs. 474-475 (I982); and Latt et al., J. Histochem. Cytochem., Vol. 25, pgs. 927-934 (I977).

More recently immunoassays for DNA substituted with bromodeoxyuridine (BrdU) and iododeoxyuridine (IdU) have been used more and more in place of assays using radioactive labels, particularly in such applications as the quantification of cell cycle kinetics, the detection of unscheduled DNA synthesis, and the isolation of naissant DNA, e.g. Gray, Cytometry, Vol. 6, pgs. 499-662 (I985); and Cohn, Biological Chemistry, Vol 259, pgs. I2456-I2462 (I984). Several monoclonal antibodies suitable for these assays have been reported, e.g. Gratzner, U. S. Patent 4,529,700); Gratzner, Science, Vol. 2I8, pgs. 474-475 (I982); Vanderlaan, Cytometry, Vol. 6, pgs. 50I-505 (I985); Traincard et al, Ann. Immunol., Vol. I34, pgs. 399-405 (I983); Gonchoroff et al, Cytometry, Vol. 6, pgs 506-505 (I985); and Raza et al, New England J. Med., Vol. 3I0, pgs. 99I-99I (I984). In all cases the immunogen used in manufacturing the antibodies was the halogenated ribouracil, conjugated to a carrier protein using the periodate/borohydride method developed by Ehrlanger et al, disclosed in Proc. Natl. Acad. Sci., Vol. 52, pgs. 68-74 (I985). This conjugation method destroys the ribose sugar, and limits the epitope to the halogenated pyrimidine. The choice of halogen appears not to influence antibody specificity, since, where reported, the resulting antibodies cross reacted highly with both IdU and BrdU.

For a number of experiments, such antibodies are unsuitable. For example, it can be shown that the monoclonal antibodies IU-I (Vanderlaan, Cytometry, Vol. 6, pgs. 50I-506 (I985)) and B-44 (Gratzner, Science, Vol. 2I8, pgs. 474-475 (I982)) require a high local linear density of BrdU substitution in the DNA strand in order to bind. The effect is that the magnitude of the fluorescence intensity depends both on the total amount of incorporated BrdU and on the spacing between BrdU residues. High linear density is readily obtained for assays on cell lines grown in vitro, but is harder to achieve when tissues are labeled in vivo. Factors such as the unequal distribution of the BrdU to the tissues and differences in endogenous thymidine monophosphate pools influence the effectiveness of BrdU labeling and therefore the strength of the immunofluorescence signal. Moreover, in clinical applications it is important to limit the dose of BrdU to minimize toxic side effects, e.g. Bloch, ed., "Chemistry, Biology, and Clinical Uses of Nucleoside Analogs," Annals of the New York Academy of Sciences, Vol. 255 (I975). But lower doses mean lighter substitution with BrdU which, in turn, means greatly reduced sensitivity.

Another disadvantage of presently available anti-BrdU monoclonal antibodies is the necessity for harsh denaturing conditions to produce enough single stranded-DNA in cells for antibody recognition of the incorporated BrdU. These conditions often result in significant cell loss, making analysis of heterogeneous cell samples, particularly from hematopoietic tissue, difficult. The conditions also denature cell surface antigens, requiring special procedures for double antibody labeling of cells for incorporated BrdU and cell surface phenotype, e.g.Houck, Cytometry, Vol. 6, pgs. 53I-538 (I985).

Both of these problems could be overcome by an antibody with higher affinity. Accordingly, an object of the present invention is to provide a high affinity monoclonal antibody to halogenated nucleosides, BrdU and IdU, incorporated into DNA.

Another object of the inventor is to provide a monoclonal antibody which permits detection of BrdU or IdU incorporated into DNA at low densities.

Still when another object of the invention is to provide a monoclonal antibody specific for BrdU or IdU incorporated into DNA, whose affinity does not depend on the density of the incorporated halogenated nucleosides.

2

Additional objects, advantages and novel features of the invention will be set forth in part in the description which follows, and in part will become apparent to those skilled in the art upon examination of the following or may be learned by practice of the invention. The objects and advantages of the invention may be realized and attained by means of the instrumentalities and combinations particularly pointed out in the appended claims.

## SUMMARY OF THE INVENTION

The invention includes monoclonal antibodies against halodeoxyuridine incorporated into DNA, and a hybridoma producing such antibodies. The hybridoma of the invention is referred to herein as IU-4, and is deposited with the American Type Culture Collection under accession number HB8916.

It is well known that antibodies comprise an assembly of polypeptide chains linked together by disulfide bridges. Two major polypeptide chains referred to as the light chain and the heavy chain, make up all major structural classes of antibody. within each heavy chain and light chain there are regions, called "variable regions," which are responsible for the antibody's binding specificity. The rest of the antibody molecule is divided into several additional regions referred to as "constant regions."

The present invention is based on the discovery of the unique variable regions possessed by the antibodies of the hybridoma IU-4. It is clear that the production (for example, by transfectoma technology, Morrison, Science, Vol. 229, pgs. 1202-1207 (1985)) of monoclonal antibodies having substantially identical variable regions, but different constant regions, would not alter the major functional attribute of the antibody of the invention, which is to bind specifically to incorporated halodeoxyuridines. Accordingly, the invention includes monoclonal antibodies having variable regions substantially identical to those of the monoclonal antibodies produced by the hybridoma IU-4, regardless of the isotype, or the origin of the constant regions. As used herein the term "variable region" refers to the sections of either the heavy chain or the light chain consisting of the sequence of 110 amino acids comprising the N-terminal end of the respective light or heavy chains. "Substantially identical" in reference to the sequences making up variable regions means that the sequences differ in length or sequence by no more than one or two amino acids, and that the regions confer substantially the same binding specificity and affinity as those of the monoclonal antibody produced by hybridoma IU-4.

Hybridoma IU-4 was derived using a different immunogen than in the prior art. Iododeoxyuridine 5'-monophosphate (IdUMP) was linked via the phosphate group to a carrier protein for immunization. Thus the immunogen presented to the immune system included not only the halogenated base, but also the correct sugar and 5' phosphate group which are present in the eventual target epitope, BrdU or IdU-subsituted DNA. A further effort was made to select a hybridoma secreting a high affinity antibody by screening the fusion colonies for antibodies recognizing substituted single stranded-DNA, thereby requiring monoclonal antibody binding even when there was wide spacing between epitopes.

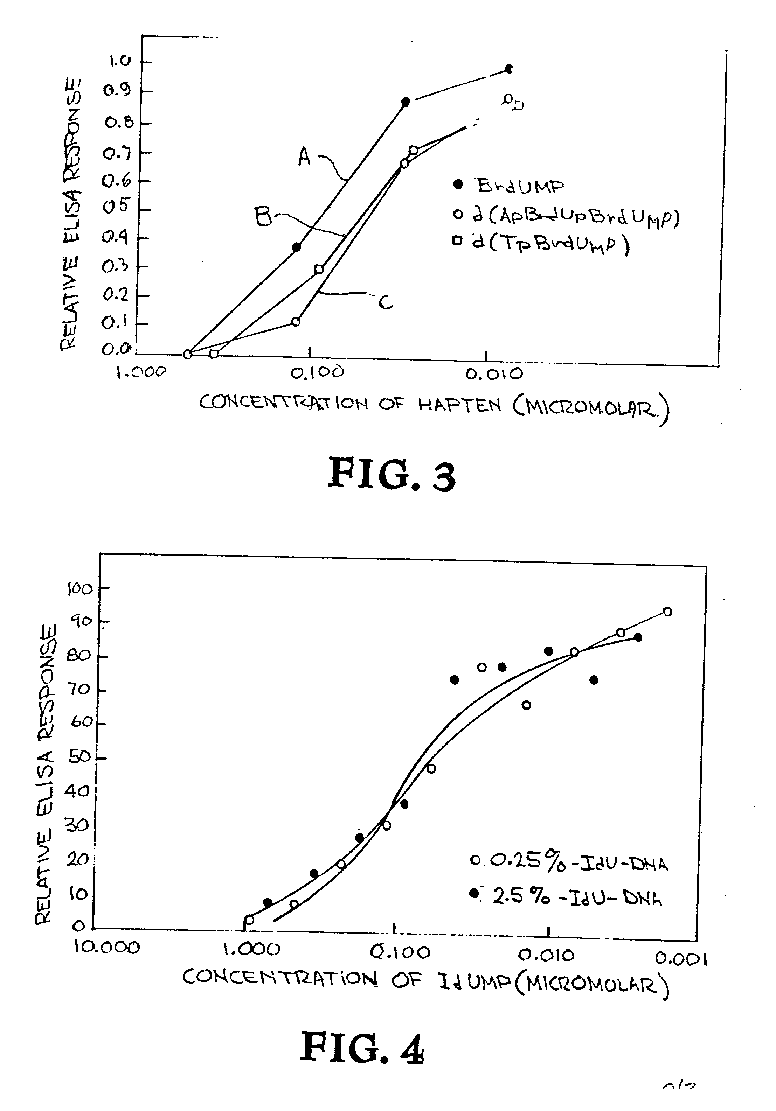

The monoclonal antibodies of hybridoma IU-4 overcome deficiencies of the prior technology by displaying an affinity for incorporated halodeoxyuridines which is several orders of magnitude greater than currently available monoclonal antibodies (e.g., see Figure I), and which does not depend on the density of incorporated halodeoxyuridines (e.g., see Figures 3-5). Hybridoma IU-4 was discovered only after several years of cell fusion and screening experiments, which suggests that it is an exceedingly rare fusion product of the parental cell lines.

## BRIEF DESCRIPTION OF THE DRAWINGS

Figure I graphically illustrates the results of direct-binding ELISA assays of prior art antibodies (IU-I) to 25 ng of HPLC single stranded 12.5%-BrdU-substantial DNA (curve A), and of antibodies from IU-4 to 125 ng of HPLC single stranded 2.5%-BrdU-substituted DNA (curve B).

Figure 2 graphically illustrates relative degrees of IU-4 antibody binding in competition ELISA assays as function of nucleotide or nucleoside concentration.

Figure 3 graphically illustrates relative degrees of IU-4 antibody binding in competition ELISA assays as functions of BrdUMP or BrdU-containing oligonucleotide concentrations.

Figure 4 graphically illustrates relative degrees of IU-4 antibody binding to DNAs having different percentages of IdU substitution.

Figure 5 graphically illustrates relative degrees of IU-4 antibody binding to DNAs having different percentages of BrdU substitution in competition ELISA assays.

Figure 6 is a contour plot of a bivariate distribution illustrating the relative frequencies of Chinese Hamster Ovary (CHO) cells with particular DNA content and amount of incorporated BrdU, the incorporated BrdU being measured by the binding of IU-4 monoclonal antibodies directly labeled with fluorescein isothiocyanate.

## DETAILED DESCRIPTION

Monoclonal antibodies of the invention are produced by culturing hybridoma IU-4 using standard culturing procedures, e.g., Schreier, et al. Hybridoma Techniques (Cold Spring Harbor Laboratory, New York, 1980); Hurrell, Ed., Monoclonal Hybridoma Antibodies: Techniques and Applications (CRC Press, Boca Raton, 1982); Campbell, Monoclonal Antibody Technology (Elsevier, New York, 1984); and Gratzner, U.S. Patent 4,529,700, which is incorporated by reference. Briefly, IU-4 can be propagated in either of two standard ways. A sample of the hybridoma can be injected into a histocompatible animal of the type that was used to provide the somatic and myeloma cells for the fusion in which the hybridoma was created, e.g., BALB/c mouse. The injected animal develops tumors secreting the specific monoclonal antibody produced by the hybridoma. The body fluids of the animal, such as serum or ascites fluid, can be tapped to provide monoclonal antibodies in high concentration. Alternatively, a hybridoma may be propagated in vitro in laboratory culture vessels; the culture medium, also containing high concentrations of the single specific monoclonal antibody, can be harvested by decantation, filtration, or centrifugation.

## I. Production of the IU-4 Hybridoma

5-Iodo-2'-deoxyuridine-5'-monophosphate (IdUMP) was coupled to bovine serum albumin (BSA) through the phosphate group using the carbodiimide procedure of Halloran et al, The Journal of Immunology, Vol. 96, pgs. 373-378 (1966). It was determined spectrophotometrically that there were 3.1 nucleotides per protein molecule.

The IU-4 hybridoma was produced by immunizing a BALB/c mouse with IdUMP-BSA conjugate. Six intraperitoneal injections were given at roughly equal intervals over a period of six months. For each injection, 100 ug of conjugate in 100 ul of water was mixed with an equal volume of Complete Freunds' Adjuvant. One additional injection of 100 ug of conjugate in sterile water without adjuvant was given intrasplenicly four days before fusion. The mouse splenocytes were removed and fused with $10^8$ SP/2O cells according to standard methods, e.g. Bigbee et al, Molec. Immun., Vol. 20, pgs. 1353-1362 (1983); Oi and Herzenberg, Selected Methods in Cellular Immunology, pgs. 351-372 (1981). Fused cells were distributed in HAT media over thirty 96-well plates and screened 14 days later in the ELISA essay described below. The colony identified as showing the greatest reactivity with BrdU-containing DNA and the least reactivity with unsubstituted DNA was selected, subcloned twice by limiting dilution and named IU-4. Large quantities of antibody were produced first in media by growing the hybridoma cells in spinner cultures, and later in ascites fluid by injecting IU-4 hybridoma cells into BALB/c mice. Antibody was purified from the ascites fluid by hydroxylapatite chromatography, e.g. Stanker et al., J. Immun. Meth., Vol. 76, pgs. 157-169 (1985). Direct conjugation of fluorescene isothiocyanate (FITC) to IU-4 inactivated the antibody unless done in the presence of ImM BrdU. Following conjugation, the BrdU was removed by dialysis.

Characterization of IU-4, was facilitated by the preparation of DNA containing BrdU or IdU at various known substitution frequencies. DNA was isolated from Chinese Hamster Ovary (CHO) cells and SP2/O cells grown in media supplemented with 10 uM 5-fluorouracil to block endogeneous thymidine synthesis by inhibition of thymidylate synthetase, 30 uM hypoxanthene, 200 uM cytidine, and various ratios of thymidine and either BrdU or Idu. $^3$H-BrdU or $^3$H-IdU (New England Nuclear, Boston) was included in the cultures to provide a basis for determining the exact amount of nucleotide incorporated into DNA. Cells were grown in these media for 3 to 4 days to allow multiple rounds of division and uniform labeling of both strands of DNA.

Following culture, the cells were pelleted and the DNA purified by lysing the cells for 3h at 4°C in buffer (0.15 M NaCl, 0.5% SDS, 10 mM EDTA pH 8) and 50 ug/ml proteinase K. Proteins were precipitated by increasing the salt concentration to 1 M NaCl, stirring for 30 min, and centrifuging for 10 min at 10,000 rpm in an ss-34 rotor (Sorval). An equal volume of ice cold 95% ethanol was layered onto the supernatant of the preceding step. DNA was purified by winding it on a glass pipette and washing twice in cold 70% ethanol. This DNA is subsequently referred to herein as "wound-DNA." The wound DNA was further purified using HPLC. The DNA additionaly purified by HPLC is termed herein "HPLC-DNA."

BrdU-labeled DNA was also produced in the B Thy- strain of E. coli developed by Clowes and obtained from the American Type Culture Collection. Cells were grown overnight in PBS-sucrose with 2 ug/ml thymidine and either 2 ug/ml of BrdU (for 12.5% substitution) or 0.2 ug/ml BrdU (for 2.5% substitution). DNA was purified by the method of Marmur, J. Mol. Biol., Vol. 3, pgs. 585-594 (1961).

Calf thymus DNA was used as a source of unsubstituted DNA.

For ELISA competition assays, wound-2.5% - IdU-single stranded DNA at 125 ng/well (10 picomolar BIdU/well) was used for the solid phase. The DNA was denatured by boiling 20-30 min., and rapidly cooled on ice, and then was dried in Immunion II microtiter plates (Dynatek, McClean, VA) which had been pre-coated with DEAE-Dextran (1.8 ng/ml in pH 9.6 0.1M carbonate buffer for 1 h). Additional binding sites on the plates were blocked with a 1% ovalbumin in pH 9.6 carbonate buffer for 1h. After blocking the plates, 100 microliters of hapten was added per well followed by 100 microliter of antibody solution. The haptens and antibodies were co-incubated in the well for 1 h. After incubation unbound antibodies and haptens were washed away, and peroxidase conjugated goat-anti-mouse immunoglobulins (US Biochemicals, Cleveland OH) were added to the wells and incubated 1-2 h. Again unbound antibodies were washed away and ABTS substrate was added to the wells. The rate of product formation was followed using a Titertek Multiscan plate reader interfaced to a computer, Slezak et al, J. Immunol. Methods, Vol. 65, pgs. 83-95 (1983). For isotyping IU-4, isotype specific antibody (Southern Biotech) used as the second antibody.

It has been determined that IU-4 secretes an IgG type antibody subclass 1 having a light chain constant regions of type kappa, e.g. see Hood et al., Chapter 3, in Immunology (Benjamin/Cummings Publishing Company, Menlo Park, CA) for an explanation of the structural classification of antibodies.

## II. Culturing and Storage of IU-4

IU-4 cells were cultured in supplemented Dulbecco's modified Eagle's medium (SDMEM) which consists of Dulbecco's modified Eagle's medium (Gibco, Santa Clara, CA) with 25 mM HEPES, 10 mM non-essential amino acids (Gioco, Santa Clara, CA), 50 micromolar 2-mercapto-ethanol, 2 mM pyruvic acid, 3 mM hypoxanthine,

300 micromolar thymidine, and 2% rabbit serum (Quandroma, Escondido, CA). The cells were maintained at 37°C in a humidified incubator with a 5% $CO_2$ in air atmosphere.

For ascites growth, BALB/c mice were injected twice with 0.2 ml pristane (e.g. available from Aldrich, Milwaukee, WI), at an interval of 1 week and irradiated with approximately 600 R of 1 MeV X-rays 1 day after the second pristane injection and approximately $10^7$ hybridoma cells were injected i.p. Typically, the mice began to die from the resulting tumors 8-15 days after injection. At this time the group of animals was sacrificed and the ascites fluids obtained by washing the peritoneal cavities with approximately 20 ml of sterile saline. Viable sterile tumor cells were recovered for reinjection into additional animals.

For storage, stocks of IU-4 may be frozen in media consisting of 70% SDMEM, 20% heat-inactivated fetal calf serum (Gibco, Santa Clara, CA), and 10% dimethylsulfoxide. In each freezing vial approximately $10^6$ cells are suspended in 0.25 ml of freezing media and cooled to -80°C at a rate of 1°C per minute. Cells are recovered by rapid thawing and seeding into pre-warmed media at a cell density of about 1-5 $\times$ $10^5$ cells/ml.

### III. Characterization of the Monoclonal Antibodies Produced by IU-4.

Antibodies from IU-4 were characterized by competition ELISA, wherein the antibodies competed for binding sites on immobilized wound-2.5%-IdU-single stranded-DNA in the presence of various concentrations of selected nucleosides, nucleotides, or oligonucleotides. The competition ELISA assays were carried out as described above.

Oligonucleotides containing bromodeoxyuridine were synthesized using a Manual DNA Synthesis Kit (Biosearch, San Rafael, CA) with modified phosphotriester chemistry. Two sequences, a trimmer d(TpBrUpA) and a tetramer d(ApBrUpBrUpA), were synthesized. The BrdU monomer phosphate, 5′-O-(dimethoxytrityl)-5-bromo-2′ deoxyuridine 3′-O-(2-chlorophenyl phosphate) triethylammonium salt, was synthesized from BrdU according to published methods, e.g., Watkins et al, J. Am. Chem. Soc., Vol. 104, pgs. 5702-5708 (1982). Other blocked monomer phosphates were purchased from Biosearch. The oligonucleotides were purified by HPLC on a C-18 reverse-phase column (Beckman) using 12% acetonitrile/0.1M triethylammonium acetate (pH=7.0) and were obtained in micromolar amounts and in greater than 98% purity. Their composition was determined by complete enzymatic digestion to nucleosides, followed by HPLC analysis (C-18 reverse-phase column, 10% methanol/0.02M sodium phosphate, pH=2.5). Both the tetramer and the trimer, were found to contain the expected composition of nucleosides.

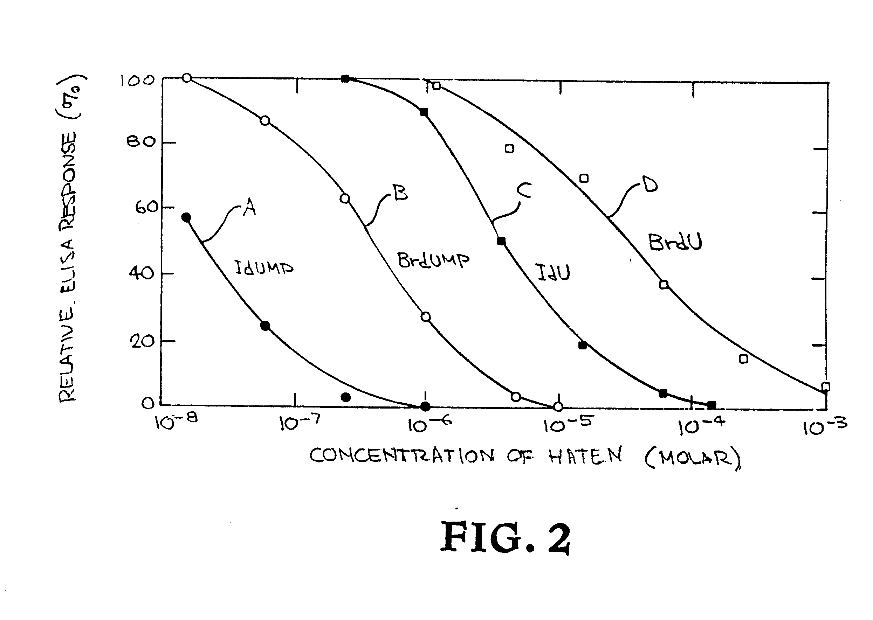

Figure 2 graphically illustrates relative degrees of antibody binding as functions of the concentrations of the indicated hapten competitors (curve A corresponds to IdUMP, curve B corresponds to BrdUMP, curve C corresponds to IdU and curve D corresponds to BrdU). The nucleotide haptens (IdUMP and BrdUMP) were preferred by the antibodies by about a factor of 100 over the corresponding nucleosides (IdU and BrdU), and iodated uracil (IdU) was preferred by about a factor of 10 over brominated uracil (BrdU).

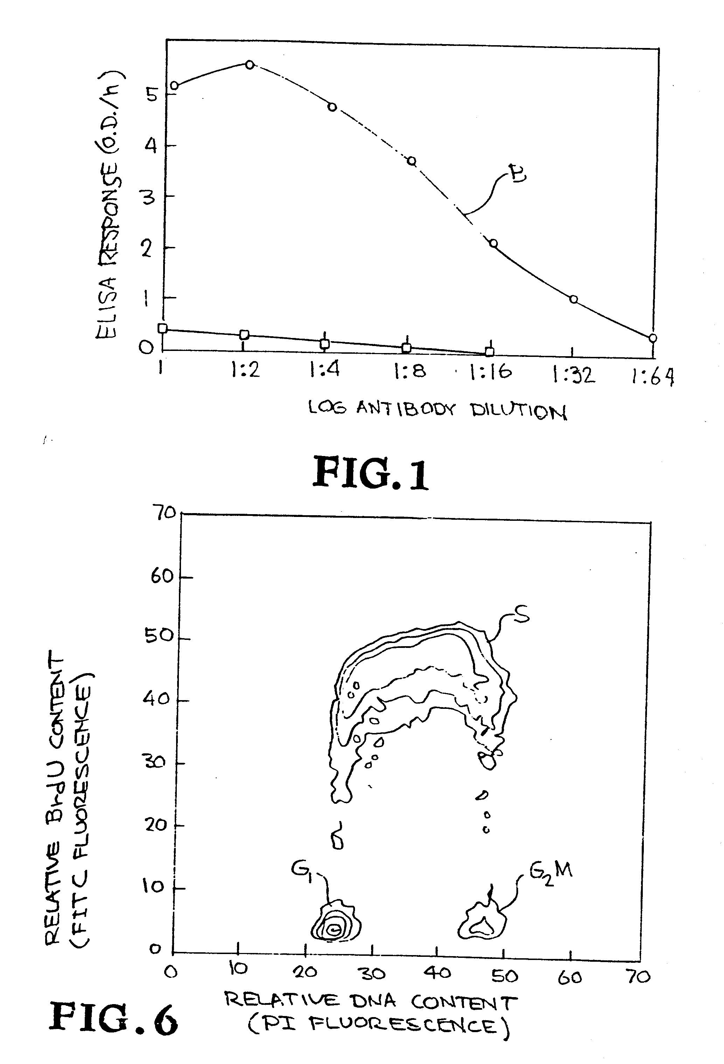

Table 1 below summarizes the information in Figure 1 and includes information on relative binding specificities (as measured by identical competition ELISA assays) for additional haptens: (5-bromo-2′-deoxyuridine-5′-monophosphate (BrdUTP); 5-iodouracil (IrU); 5-chlorodeoxyuridine (CldU); 5-fluoro-2′-deoxyuridine-5-monophosphate (FldUMP); and 2′-deoxythymidine-5′-monophosphate (dTMP).

## Table I

| Hapten | Concentrations for 50% Inhibition of Binding to Solid Phase DNA (micromolar) | Relative Binding Affinity with Respect to IdUMP (percent) |
|---|---|---|
| IdUMP | 0.02 | 100 |
| BrUMP | 0.2 | 10 |
| BrdUTP | 0.4 | 5 |
| IdU | 0.5 | 4 |
| BrdU | 1.5 | 1.3 |
| IrU | 1.5 | 1.3 |
| CldU | 6.25 | .32 |
| FldUMP | 40. | .05 |
| dTMP | 50. | .04 |

Figure 3 graphically illustrates relative degrees of IU-4 antibody binding in competition ELISA assays as functions of the following hapten competitor concentrations: BrdU (curve A), oligonucleotide d(TpBrUpA) (curve B), or oligonucleotide d(ApBrUpBrUpA) (curve C). Roughly equal binding specificity is demonstrated against each of the three competitors, showing that antibody binding is insensitive to the presence of the 3' phosphate and also insensitive to adjacent bases. It appears that there may be slightly more reactivity toward the BrdUMP in the context of the other nucleotides than as a free compound although the differences are very slight. The oligo-nucleotide containing two adjacent BrdU's was recognized no more effectively than the one containing a single BrdU, showing that the antibody does not prefer adjacent BrdU groups that might occur by chance in DNA.

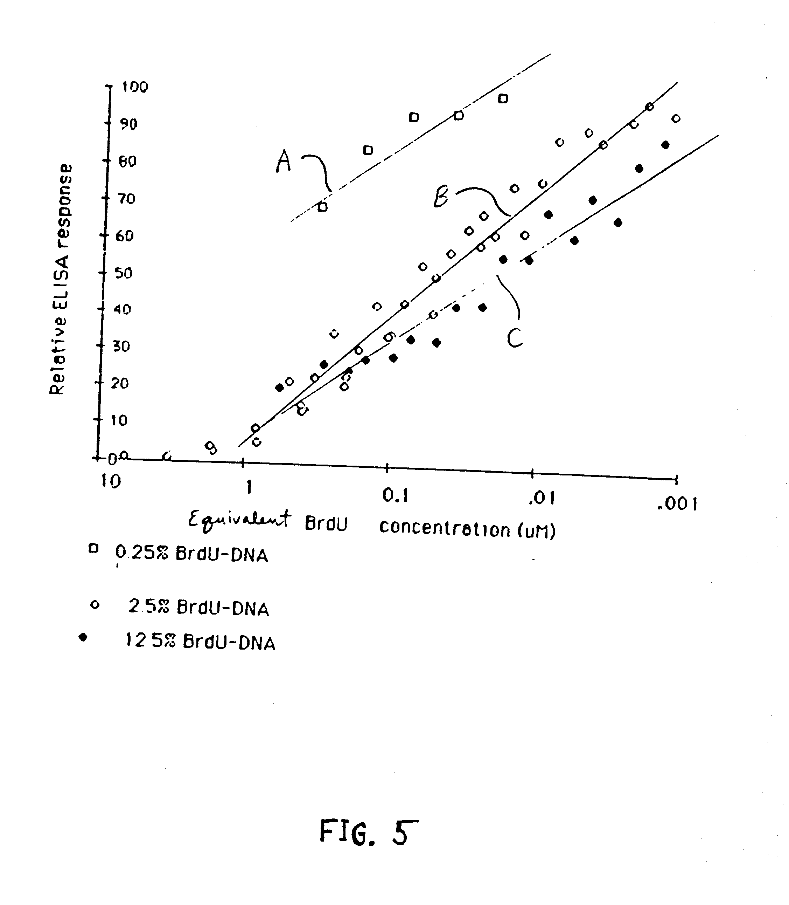

Figure 4 graphically illustrates relative degrees of IU-4 antibody binding to DNAs having different percentages (O.25 in curve A and 2.5 in curve B) of IdU substitution in competition ELISA assays. The abscissas of the graphs in both Figure 4 and Figure 5 give the equivalent concentration of iodated or brominated uracil. That is, the abscissas indicate the concentrations of BrdU or IdU binding sites collectively carried by the hapten competitors.

Figure 5 graphically illustrates relative degrees of IU-4 antibody binding to DNAs having different percentages of BrdU substitution (O.25 in curve A, 2.5 in curve B, and I2.5 in curve C) in ELISA competition assays.

The monoclonal antibodies of the invention are particularly useful in flow cytometric assays of DNA synthesis in all populations. Figure 6 is a contour plot of a bivariate distribution illustrating the relative frequencies of Chinese Hamster Ovary (CHO) cells with particular DNA content and amount of incorporated BrdU. The CHO Cells were pulse labeled with BrdU for 3O minutes, fixed, stained with monoclonal antibodies from IU-4 directly conjugated to fluorescein isothiocyanate, and then counterstained with propidium iodide, following the procedure of Dolbeare et al., Proc. Natl. Acad. Sci., Vol. 8O, pgs. 5573-5577 (I983); and Dolbeare et al., Cytometry, Vol. 6, pgs. 52I-53O (I985). Cells in the various cell cycle phases are identified on the graph.

The disclosure of the foregoing embodiment of the invention has been presented for purposes of illustration and description. It is not intended to be exhaustive or to limit the invention to the precise form disclosed, and obviously many modifications and variations are possible in light of the above teaching. The embodiment was chosen and described in order to best explain the principles of the invention and its practical application to thereby enable others skilled in the art to best utilize the invention in various embodiments and with various modifications as are suited to the particular use contemplated. It is intended that the scope of the invention to be defined by the claims appended hereto.

6

## Claims

1. A monoclonal antibody specific for halodeoxyuridine incorporated into DNA, the halodeoxyuridine being BrdU or IdU.

2. An antibody according to claim 1 wherein the affinity of said antibody does not depend on the density of BrdU or IdU incorporated into said DNA.

3. An antibody according to claim 1 or 2 wherein the heavy and light chain variable regions are substantially identical to those of the monoclonal antibody produced by hybridoma IU-4.

4. An antibody according to claim 2 whose binding specificity is substantially the same as the binding specificity defined by the data displayed in Table I and in Figures 2 to 5.

5. An antibody according to claim 1 produced by a hybridoma formed by fusion of cells from a mouse myeloma line and spleen cells from a mouse previously immunized with 5-iododeoxyuridine-5'-monophosphate conjugated via the 5'-monophosphate to a protein.

6. An antibody according to claim 1 which is produced by a hybridoma having the identifying characteristics of the cell line deposited under ATCC HB 8916.

7. An antibody according to claim 6, wherein said monoclonal antibody is produced by hybridoma IU-4 (ATCC HB 8916).

8. A hybridoma cell line which produces an antibody according to any one of the preceding claims.

9. A hybridoma according to claim 8 which has the identifying characteristics of ATCC HB 8916.

10. A method of producing monoclonal antibody specific for halodeoxyuridine incorporated into DNA, the halodeoxyuridine being BrdU or IdU, which comprises culturing a hybridoma according to claim 8 or 9 and recovering said antibody.

0239405

FIG.1

FIG.6

RELATIVE ELISA RESPONSE (%)

CONCENTRATION OF HATEN (MOLAR)

A IdUMP
B BrdUMP
C IdU
D BrdU

**FIG. 2**

FIG. 3

FIG. 4

0239405

FIG. 5